**Europäisches Patentamt**

**European Patent Office**

**Office européen des brevets**

(19)

(11) Numéro de publication: **0 251 842**
**A1**

(12) ## DEMANDE DE BREVET EUROPEEN

(21) Numéro de dépôt: **87401248.7**

(22) Date de dépôt: **03.06.87**

(51) Int. Cl.³: **C 12 N 15/00**
C 12 P 21/02, C 12 N 1/20
//(C12N1/20, C12R1:19),
(C12P21/02, C12R1:19)

(30) Priorité: **05.06.86 FR 8608139**

(43) Date de publication de la demande:
**07.01.88 Bulletin 88/1**

(84) Etats contractants désignés:
**AT BE CH DE ES FR GB GR IT LI LU NL SE**

(71) Demandeur: **SANOFI**
**40, Avenue George V**
**F-75008 Paris(FR)**

(72) Inventeur: **Bahramian, Bahman**
**4, rue Léon Viala**
**F-31520 Ramonville St. Agne(FR)**

(74) Mandataire: **Gillard, Marie-Louise et al,**
**Cabinet Beau de Loménie 55, Rue d'Amsterdam**
**F-75008 Paris(FR)**

(54) **Séquence d'ADN composite et son application pour la production de l'hormone de croissance.**

(57) La présente invention concerne une séquence d'ADN composite constituée de son extrémité 5' vers son extrémité 3' par :

a) la séquence d'ADN naturelle codant pour l'extrémité 5' non traduite de l'ARN messager de l'opéron lactose d'*E. coli* incluant la séquence de Shine et Dalgarno;

b) une séquence d'ADN ayant la structure 5' AATTATGGCTACCGGCTCT 3';

c) la séquence d'ADN naturelle correspondant à l'extrémité carboxy-terminale du peptide signal de l'hGH comptée à partir du seizième nucléotide constitutif du peptide signal.

Application : production de l'hormone de croissance.

EP 0 251 842 A1

Séquence d'ADN composite et son application pour la production
de l'hormone de croissance.

La présente invention concerne une nouvelle séquence
d'ADN composite qui, placée en amont d'un gène codant pour l'hormone
de croissance, synthétisée sous la forme d'un précurseur, et en
aval du promoteur sous le contrôle duquel est placé ledit gène,
favorise dans une bactérie l'expression, la sécrétion et la maturation correcte de ladite hormone.

Les progrès du génie génétique ont permis de pallier
les inconvénients des méthodes classiques d'obtention de protéines
d'origine eucaryote susceptibles de constituer les principes
actifs de nombreux médicaments.

Ces méthodes faisant appel notamment à des extractions
à partir de liquides physiologiques ou d'organes, les médicaments
qui en étaient issus n'étaient pas produits en quantité suffisante
et surtout n'étaient pas toujours de qualité constante.

Les techniques du génie génétique permettent maintenant
d'obtenir une synthèse protéique par la mise en oeuvre de micro-
organismes : pour cela, on insère dans le génome d'un micro-organisme
un gène provenant d'un autre micro-organisme et on fait s'exprimer
ce gène cloné.

Certaines protéines sont synthétisées sous la forme
d'un précurseur. Ce précurseur correspond à la protéine elle-même
allongée au niveau de son extrémité N-terminale d'une courte séquence
d'acides aminés appelée peptide signal. Il est soumis à une exportation du milieu cellulaire où il a été synthétisé vers le milieu
extra-cellulaire ou le périplasme dans le cas des bactéries gramnégatives; cette exportation se caractérise par le franchissement
de la membrane cytoplasmique au cours duquel, par l'action d'une
enzyme, le peptide signal et la protéine mature sont séparés; la
protéine mature étant recueillie dans le milieu extra-cellulaire
ou à partir du périplasme.

La présente demande de brevet est relative à l'obtention
de l'hormone de croissance qui est synthétisée sous forme d'un
précurseur et plus particulièrementde l'hormone de croissance humaine
(ci-après désignée hGH) laquelle comporte 191 acides aminés et est synthétisée sous la forme d'un précurseur de 217 acides aminés, (ci-après désigné pré-hGH).

De nombreux vecteurs d'expression bactériens jusqu'ici obtenus se sont avérés peu appropriés à une production industrielle significative de protéines d'origine eucaryote matures synthétisées sous la forme d'un précurseur.

Par exemple, seules de très faibles quantités d'insuline ont pu être obtenues (K. TALMADGE et al, Bacteria mature preproinsulin to proinsulin, Proc. Natl. Acad. Sci., USA, 1980,77, 3988-3992). De même, de l'interféron d'origine fibroblastique humain a pu être obtenu mais il ne présentait pas d'activité biologique (T. TANIGUCHI et al., Expression of the human fibroblast interferon gene in Escherichia coli,Proc. Natl. Acad. Sci.,USA, 1980, 77, 5230-5233).

G. GRAY et al (Periplasmic production of correctly processed human growth hormone in Escherichia coli : natural and bacterial signal sequences are interchangeable, Gene, 1985, 39, 247-254) ont décrit la production d'hGH périplasmique dans E. coli. Il faut noter que,dans ce cas, la séquence codant pour le précurseur de l'hGH est placée sous le contrôle du promoteur tryptophane. Bien que ce promoteur possède la caractéristique particulière de régulation de la transcription d'autres promoteurs qu'est l'inductibilité, il est difficile de contrôler son activité pour l'expression de gènes étrangers.

L'activité du promoteur-opérateur de l'opéron lactose(ci-après désigné P/O lac) qui est également inductible peut être par contre aisément maîtrisée.En l'absence de lactose, le répresseur spécifique de l'opéron lactose, codé par le gène i, se fixe sur la séquence opérateur de l'opéron et fait obstacle à l'initiation de la transcription par l'ARN polymérase. En présence de lactose ou d'un autre inducteur auquel vient se fixer le répresseur, la transcription peut être normalement initiée.

Il est connu que le rendement de la synthèse d'une protéine dépend de l'efficacité :

- de la transcription du gène qui lui correspond, et

- de la traduction de l'ARN messager (ci-après désigné ARNm) ainsi produit.

La transcription se fait grâce à l'intervention d'une enzyme, l'ARN polymérase qui se fixe en une région spécifique appelée le promoteur. Après s'être fixée au promoteur, l'ARN polymérase initie la transcription de l'ADN consécutivement à laquelle de l'ARNm est obtenu.

La traduction est réalisée grâce à l'intervention des ribosomes qui viennent se fixer sur l'ARNm au niveau d'un site de fixation proche de son extrémité 5'.

Quelques éléments du site de fixation des ribosomes sont connus : c'est une séquence d'ARNm constituée du codon d'initiation AUG (c'est le premier codon traduit) et d'une séquence appelée séquence de Shine et Dalgarno (ci-après désignée séquence S/D) située à environ dix nucléotides en amont du codon AUG. La séquence S/D qui comprend de 3 à 9 nucléotides permet à l'ARNm d'être reconnu par l'ARN ribosomal 16S (ci-après désigné ARNr 16S) dont l'extrémité 3' est complémentaire de la séquence S/D.

Il est également connu que la synthèse d'une protéine dépend surtout de l'efficacité de l'initiation de la traduction de l'ARNm, qui est elle-même fonction de la séquence S/D et de la distance séparant cette séquence du codon d'initiation.

Cette initiation fait intervenir de multiples paramètres :

- Il a été ainsi montré (H.M. SHEPARD et al., DNA, 1982, 1, 125-131) que la distance entre la séquence S/D et le codon d'initiation affectait l'efficacité de la traduction, la distance optimale étant de neuf nucléotides.

- De même, il est admis que plus la complémentarité de la séquence S/D et de l'ARNr 16S est grande, plus forte est la fixation du ribosome, bien qu'il n'ait pas encore été trouvé de relation simple entre l'efficacité de la traduction d'un ARNm particulier et sa complémentarité au regard de l'ARNr 16S (G.D. STORMO et al., Nucl. Acids. Res., 1982, 10, 2971-2996).

- Il est aussi admis que les bases "adenine" et "thymine" placées entre la séquence S/D et le codon AUG favorisent la traduction (DE BOER et al., DNA, 1983, 2, 231-235).

- Il a été postulé qu'une séquence optimale ne suffit pas à assurer l'efficacité de la traduction, encore faut-il que le codon d'initiation et/ou la séquence S/D soient bien accessibles aux ribosomes et non pas masqués par la structure secondaire que pourrait prendre l'ARNm (D. GHEYSEN et al.; Gene, 1982, 17, 55-63.)

Toutefois l'utilisation de tous les paramètres décrits ne peut pas assurer un bon rendement de la synthèse protéique.

On a maintenant trouvé que des vecteurs contenant une nouvelle séquence d'ADN composite pouvaient être efficacement utilisés pour l'obtention d'une protéine mature synthétisée sous forme d'un précurseur.

La nouvelle séquence d'ADN composite est constituée, de son extrémité 5' vers son extrémité 3' par:

- l'ADN qui code pour l'extrémité 5' non traduite de l'ARN messager de l'opéron lactose de E. coli (The Operon : Miller J.H. and TEZNIKOFF W.S., Cold Spring Harbor Laboratory, 1980) qui inclut la séquence S/D.
- un oligonucléotide ayant la structure 5' AATTATGGCTACCGGCTCT 3',
- la séquence naturelle d'ADN codant pour les 21 acides aminés du peptide signal de l'hormone de croissance humaine, compris entre l'acide aminé en position-21 et l'acide aminé en position-1.

Il est apparu effectivement que la structure tridimensionnelle que prend l'extrémité 5' de l'ARNm,résultant de la structure définie ci-dessus et codant pour le peptide signal, joue un rôle fondamental pour la fixation du ribosome et l'initiation de la traduction.

Ainsi,selon l'un de ses aspects, l'invention concerne une séquence d'ADN composite pour la synthèse de l'hormone de croissance contenant,de son extrémité 5' vers son extrémité 3':

- l'ADN qui code pour l'extrémité 5' non traduite de l'ARN messager de l'opéron lactose de E. coli qui inclut la séquence S/D;
- un oligonucléotide ayant la structure 5' AATTATGGCTACCGGCTCT 3'. Cette séquence inclut quatre nucléotides en amont du codon d'initiation de la synthèse protéique (ATG) et douze nucléotides en aval d'ATG, correspondant à la séquence d'ADN codant pour la partie N—terminale du peptide signal, dans laquelle deux codons ont été modifiés : ACA en ACC et TCC en TCT.

- la séquence naturelle d'ADN correspondant à l'extrémité C terminale du peptide signal de l'hormone de croissance humaine compté à partir du seizième nucléotide constitutif du peptide signal, à savoir la séquence d'ADN qui code pour les 21 acides aminés du peptide signal entre l'acide aminé en position-21 et l'acide aminé en position-1.

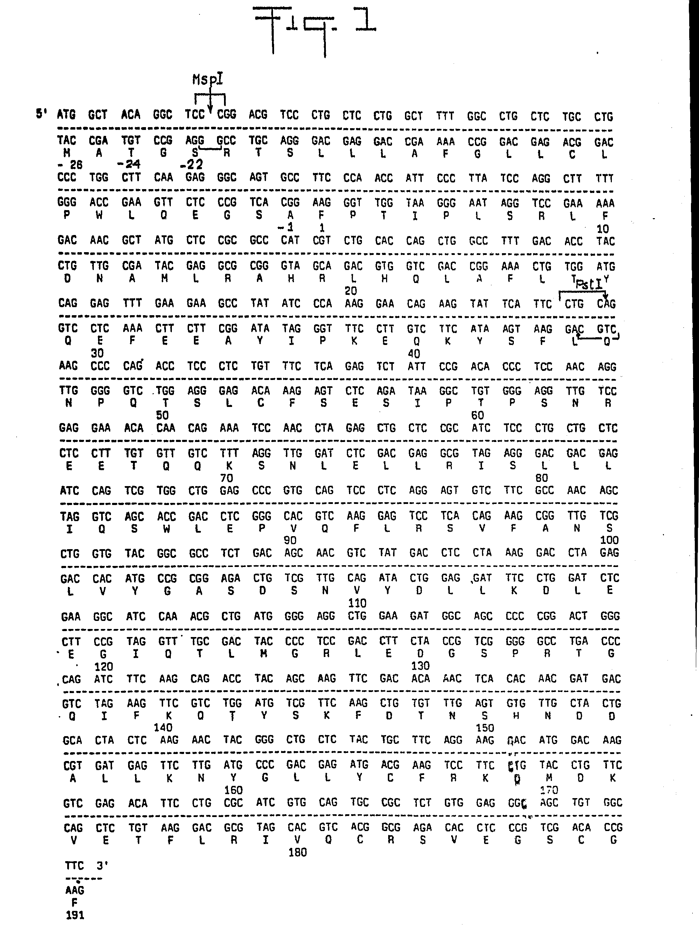

La figure 1 annexée représente la séquence de l'ADN codant pour le précurseur de l'hGH ainsi que la séquence des acides aminés dudit précurseur : les 26 premiers acides aminés (comptés sur la figure à partir de l'acide aminé en position-26) constituent le peptide signal et les 191 autres acides aminés (ici numérotés de 1 à 191) sont ceux de l'hGH proprement dite.

Le tableau No. 1 ci-après est ici inséré pour permettre une meilleure compréhension de la figure 1 où chacun des acides aminés est représenté par une seule lettre.

### Tableau No. 1

| Acides aminés | Lettre |
|---|---|
| Alanine | A |
| Arginine | R |
| Asparagine | N |
| Acide aspartique | D |
| Cystéine | C |
| Glutamine | Q |
| Acide glutamique | E |
| Glycine | G |
| Histidine | H |
| Isoleucine | I |
| Leucine | L |
| Lysine | K |
| Méthionine | M |
| Phénylalanine | F |
| Proline | P |
| Sérine | S |
| Thréonine | T |
| Tryptophane | W |
| Tyrosine | Y |
| Valine | V |

0251842

6

Dans les codons de la séquence d'ADN représentée sur la figure 1, les lettres A, C, T et G ont la signification habituelle et désignent respectivement les bases : adénine, cytosine, thymine et guanine.

L'invention concerne également les séquences d'ADN composites dans lesquelles ont été remplacées une ou deux bases dudit oligonucléotide dans la mesure où la fonction de ladite séquence n'est pas modifiée.

La séquence d'ADN composite placée sous le contrôle d'un promoteur produit des molécules d'ARNm dont le site d'attachement du ribosome adopte une structure linéaire favorisant la traduction desdites molécules.

La séquence d'ADN composite selon l'invention est placée en amont du gène codant pour l'hormone de croissance humaine, les nucléotides de cette séquence qui codent pour l'extrémité N-terminale du peptide signal ayant remplacé ceux normalement apportés par le gène codant pour le précurseur de l'hGH.

Selon un autre de ses aspects, l'invention concerne un vecteur d'expression bactérien comportant ladite séquence objet de l'invention, placée entre un promoteur bactérien et une séquence d'ADN codant pour l'hGH.

Selon un autre aspect, l'invention concerne les plasmides contenant une séquence d'ADN codant pour l'hormone de croissance humaine et comprenant une séquence d'ADN composite selon l'invention et un promoteur en amont de cette séquence. Trois plasmides préférés sont les plasmides p149-147, p169-30, et p171-6 définis ci-après. Le plasmide p149-147 a fait l'objet le 26 février 1986 d'un dépôt dans la souche d'E. coli C 1024 auprès de la Collection Nationale de Cultures de Micro-organismes (C.N.C.M. Paris-France) qui lui a attribué le numéro I-533.

L'invention concerne également les micro-organismes transformés par un vecteur selon l'invention et notamment ceux appartenant à la famille des Enterobacteriaceae.

Elle concerne plus particulièrement l'espèce Escherichia coli et, à titre d'exemple, la souche C1024.

Selon un autre aspect, l'invention concerne un procédé pour l'obtention de l'hormone de croissance humaine par culture d'un micro-organisme recombiné, suivie de l'extraction de l'hormone de la cellule, par exemple par choc osmotique et son isolement par purification du chocat (milieu extra-cellulaire après le choc), dans lequel le micro-organisme recombiné mis en oeuvre est l'un des micro-organismes selon l'invention.

Les plasmides contenant une séquence d'ADN composite selon l'invention sont construits à partir de plasmides apportant les éléments constitutifs ; ces plasmides étant découpés en fragments par digestions enzymatiques et les fragments d'intérêt étant réunis par ligation enzymatique. La séquence d'ADN composite peut être obtenue par voie de synthèse. Elle est alors traitée comme les fragments ci-dessus.

La présente invention est illustrée par les exemples suivants. L'invention bien entendu ne se limite pas aux modes d'application ici plus particulièrement envisagés, mais elle en embrasse au contraire toutes les variantes dans le cadre des revendications.

1- METHODOLOGIE GENERALE

Toutes les opérations nécessaires pour la construction de plasmides contenant une séquence d'ADN composite selon l'invention font appel à des techniques générales décrites dans l'art antérieur et des techniques particulières qui sont par commodité indiquées ci-dessous.

PURIFICATION DES PLASMIDES.

Les plasmides ont été isolés des cellules d'E. coli transformées, cultivées en milieu liquide de Luria (Milieu L) (T. MANIATIS, et al., p. 88 et 410, in Molecular Cloning : A Laboratory Manual, Cold Spring Harbor Press, New York, N.Y. 1982). Les plasmides ont été isolés des cellules en phase exponentielle tardive de croissance, en utilisant une modification de la méthode de L. Katz et al., 1973, J. Bact. 114 : 557-591. Les plasmides sont d'abord soumis à deux centrifugations à l'équilibre en gradient de chlorure de césium/bromure d'éthidium, suivies par des extractions au butanol. Ils sont ensuite purifiés par 2 cycles d'extraction au mélange chloroforme - alcool isoamylique (24:1), et précipitation par 2 volumes d'éthanol absolu froid additionné de 0,1 volume de NaCl 3 M. L'ADN précipité a été lavé une fois avec de l'éthanol à 70% et une fois avec de l'éthanol absolu, puis séché sous vide.

DIGESTION PAR LES ENZYMES DE RESTRICTION :

Les enzymes de restriction utilisés dans la présente invention sont disponibles auprès de New England Biolab. Inc. U.S.A. Une unité d'enzyme est définie comme la quantité nécessaire pour digérer 1,0 µg d'ADN de bactériophage lambda en 60 min à 37°C, dans un volume réactionnel de 50 µl.

Les digestions totales sont effectuées de la façon suivante: 1 µg d'ADN est incubé avec 2 à 5 unités d'enzyme à 37°C pendant 60 min dans 20 µl de tampon.

En ce qui concerne les digestions partielles, 1,5 µg d'ADN est incubé avec 0,75 unité d'enzyme dans 10 µl de tampon à 37°C.

A différents intervalles de temps, 1 µl d'échantillon est prélevé et mélangé à 4 µl d'une solution contenant du glycérol, de l'EDTA et du bleu de bromophénol afin d'obtenir un échantillon contenant :

20 mM d'EDTA (Acide éthylènediaminetétraacétique), 10% de glycérol, 0,025% de bleu de bromophénol.

Les différents échantillons sont soumis à une électrophorèse sur gel d'agarose. Après migration et coloration des fragments d'ADN au bromure d'éthidium, les fragments sont visualisés sous ultraviolets et on détermine le temps optimal pour obtenir une quantité maximale d'un fragment particulier d'ADN.

Les tampons utilisés ont la composition suivante :

. Pour les digestions ClaI et TaqI : tri (hydroxyméthyl) amino méthane HCl (Tris HCl) 10 mM (pH 7,5), MgCl$_2$ 10 mM, DTT (dithiothréitol) 1 mM, 100 µg/ml de gélatine.

. Pour la digestion EcoRI : Tris HCl 100 mM (pH 7,6) MgCl$_2$ 5 mM, NaCl 50 mM, 100 µg/ml de gélatine.

. Pour la digestion MspI : Tris HCl 10 mM (pH 7,6), .
. MgCl$_2$, 10 mM, KCl 6 mM, DTT 1 mM, 100 µg/ml de gélatine.

. Pour les digestions AluI, BamHI, HindIII, HinfI ou PvuII : Tris-HCl 10 mM (pH 7,5), MgCl$_2$ 10 mM, DTT 1 mM, 100 µg/ml de gélatine.

. Pour la digestion PstI : Tris HCl 10 mM (pH 7,6), MgCl$_2$ 6mM, NaCl 50 mM, DTT 1 mM, 100 µg/ml de gélatine.

PURIFICATION DES FRAGMENTS D'ADN :

Après avoir subi l'action des enzymes de restriction, les fragments d'ADN de différentes tailles sont séparés par électrophorèse sur gel d'agarose ou de polyacrylamide (5÷10 V/cm), colorés au bromure d'éthidium et visualisés sous UV (T. MANIATIS, et al. , Molecular Cloning : A Laboratory Manual, 1982, Cold Spring Harbor Press, New York, NY, 150-178).

Afin de récupérer les fragments d'ADN particuliers sur le gel, les bandes d'intérêt sont découpées, et l'ADN est électro-élué dans des boudins de dialyse.

L'ADN est ensuite isolé sur une colonne de DEAE-cellulose, précipité à l'éthanol et remis en suspension dans le tampon approprié (HO SMITH, Methods in Enzymology, 1980, 65, 371-380).

CONSTRUCTION DES OLIGONUCLEOTIDES DOUBLE BRIN :

Deux oligonucléotides monobrin de 19 nucléotides (5'AATTATGGCTACCGGCTCT) et 17 nucléotides (5'CGAGAGCCGGTAGCCAT) ont été synthétisés.

Des quantités equimolaires de chaque oligonucléotide. (environ 2 µg) sont mélangées dans un tube Eppendorf stérile et chauffées à 70°C pendant 2-3 minutes. Elles sont ensuite refroidies à température ambiante puis l'extrémité 5' des oligonucléotides est phosphorylée par addition de 2 µl d'un tampon "kinase" dix fois concentré (500 mM de Tris HCl, pH 7,8 : 100 mM de MgCl$_2$, 50 mM de DTT), de 2 µl de 10 mM rATP et de 1 µl de polynucléotide kinase de bactériophage T4 (titrant 10 U/ml, laboratoires BRL), ceci dans 20 µl de volume réactionnel à 37°C pendant 45 min. La "kinase" est inactivée par chauffage du mélange à 90°C pendant 5 min. Le mélange est ensuite refroidi doucement à température ambiante, toute une nuit afin de permettre aux oligonucléotides de s'hybrider.

LIGATION DE L'ADN

Les ligations d'ADN sont accomplies en utilisant l'ADN-ligase du bactériophage T4 (New England Nuclear). La composition du tampon de ligation est la suivante :
50 mM de Tris HCl (pH 7,8), 10 mM de $MgCl_2$, 20 mM de DTT, 1,0 mM d'ATP, 50 µg/ml de gélatine pour une concentration en ADN variant de 15 à 50 µg/ml. La réaction a lieu à 10°C pendant 17 à 24 heures et en utilisant environ 8 unités de l'ADN-ligase du bactériophage T4 pour 20 µl de volume réactionnel. Ces ligations permettent l'obtention de plasmides contenant la séquence d'ADN composite selon l'invention.

TRANSFORMATION DES CELLULES HOTES

Les cellules d'E. coli sont rendues perméables aux plasmides et aux fragments d'ADN comme suit :
2 ml de culture de cellules sur milieu L dans une phase de croissance exponentielle présentant une absorbance mesurée à 600 nm égale à 0,3 sont refroidis sur glace.

Les cellules sont récoltées par centrifugation à 4°C (5000 tr/min, 3000 g) elles sont doucement resuspendues dans 1,2 ml d'une solution stérile de $CaCl_2$ (50 mM) glacée. Après 10 min d'incubation dans la glace, les cellules sont récupérées par une centrifugation et le culot de cellules est resuspendu dans 200 µl de 50 mM $CaCl_2$ froid et incubé à 4°C pendant 20-24 heures afin de produire des cellules compétentes.
200 ng de l'ADN lié (dans environ 90 µl de tampon de ligation) sont mélangés doucement avec les cellules compétentes. Le mélange est transféré dans la glace pendant 10 min, puis placé au bain-marie à 37°C sans agitation pendant 5 min.

500 µl du milieu L sont ajoutés au tube, le mélange est mis sans agitation à 37°C pendant 50 min, puis est étalé sur 4 boîtes de milieu L additionné d'agar contenant 100 µg/ml d'ampicilline. Les colonies résistantes à l'ampicilline contiennent les plasmides recombinés (qui par commodité pour la sélection portent le gène amp[r] codant pour la bêta-lactamase qui confère la résistance à l'ampicilline).

2 - RESULTATS

3 plasmides contenant une séquence d'ADN composite selon l'invention ont été construits.

EXEMPLE 1 : CONSTRUCTION DU PLASMIDE p149-147

La construction du plasmide p149-147 résulte de la prise en considération des trois paramètres suivants :

- La distance sur l'ARNm séparant la séquence S/D du codon AUG doit être optimale, c'est-à-dire égale à 9 nucléotides,

- des adénines et des thymines de préférence à des cytosines ou des guanines doivent être placées autour de la séquence d'ADN codant pour la séquence S/D et de part et d'autre du codon ATG,

- il est avantageux de modifier par substitution d'un nucléotide les triplets ACA et TCC codant respectivement pour les acides aminés du peptide signal placés en position—24 et —22 (ACA est remplacé par ACC et TCC par TCT) (voir figure 1).

Le plasmide p149-147 est obtenu par l'association :

- d'un fragment d'un plasmide repéré par la référence p46-6 (fragment MspI-PstI),

- de deux fragments d'un plasmide repéré par la référence p126-2 (fragment PstI-PstI et fragment PstI-EcoRI), et,

- d'un oligonucléotide double brin de synthèse.

1.1. Construction du plasmide p46-6

Le plasmide p46-6 est construit à partir des plasmides p40-4 et plac2.

a) Construction du plasmide p40-4 (figure 2)

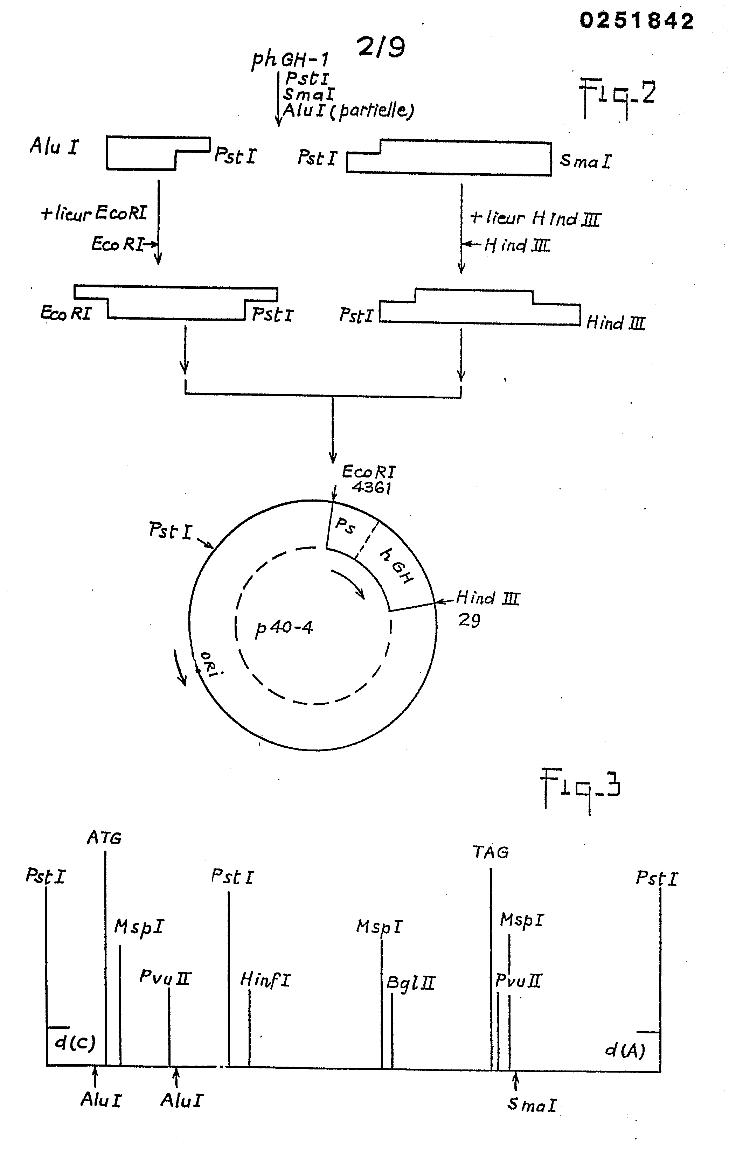

Cette construction a pour point de départ le plasmide phGH-1 décrit par W. ROSKAM et F. ROUGEON, dans Nucl. Acids. Res., 1979, 7, 305-320. La figure 3 présente les positions respectives de certains sites de restriction du plasmide phGH-1.

Ce plasmide phGH-1 porte la séquence naturelle d'ADN codant pour le précurseur de l'hGH. Cette séquence est comprise entre les deux sites, marqués d'une flèche, AluI (situé 5 nucléotides en amont du codon ATG codant pour l'acide aminé en position -26 du peptide signal) et SmaI (situé 6 nucléotides en aval du codon TAG codant pour la terminaison de la traduction de ladite séquence).

Le plasmide p40-4 résulte de l'insertion dans le plasmide pBR322 de deux fragments du plasmide phGH-1 selon le mode opératoire ci-après :

Les sites de restriction AluI (5 nucléotides en amont d'ATG) et SmaI (6 nucléotides en aval d'TAG) sont choisis pour isoler la séquence codant pour le précurseur de l'hGH.

Après digestion complète de phGH-1 par les enzymes SmaI et PstI et partielle par l'enzyme AluI, deux fragments sont isolés : AluI-PstI (200 paires de bases) et PstI-SmaI (450 paires de bases).

Les sites AluI et SmaI sont modifiés en sites EcoRI et Hind III respectivement en utilisant des lieurs EcoRI et HindIII appropriés. Après coupure par les enzymes de restriction EcoRI et HindIII, les nouveaux fragments EcoRI - PstI et PstI - HindIII sont clonés indépendamment dans le plasmide pBR322. Les plasmides ainsi obtenus sont clonés dans des cellules de la souche d'E.coli C. 600 et amplifiés.

Lesdits fragments sont ensuite purifiés à partir de ces plasmides et sont insérés ensemble dans la même grande séquence EcoRI—HindIII de pBR322, à savoir entre les sites EcoRI 4361 et HindIII 29.

Les différentes étapes ci-dessus sont représentées schématiquement sur la figure 2, sur laquelle :
- PS désigne la séquence d'ADN codant pour le peptide signal de l'hGH, et
- hGH désigne la séquence codant pour l'hGH.
L'ensemble PS-hGH désigne la séquence codant pour le précurseur de l'hGH.

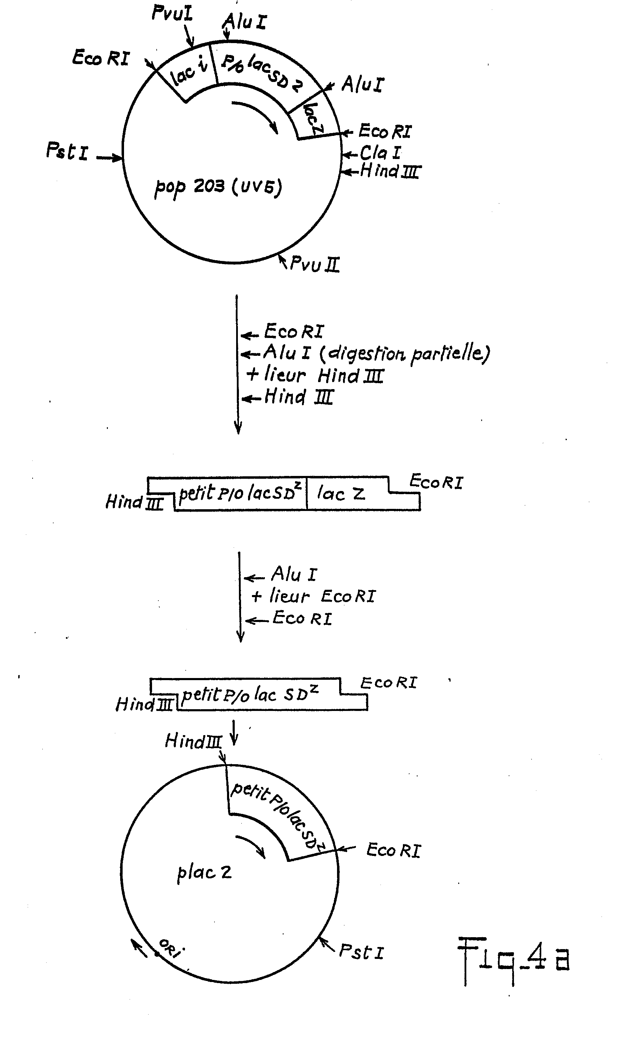

## b) Construction du plasmide plac2  (Figure 4a)

Le plasmide plac2  a été obtenu à partir du plasmide pOP203(UV5) décrit par P. CHARNAY et al. dans Nucl. Acids Res., 1978, 5, 4479-4494; K. BACKMAN et al., Proc. Natl. Acad. Sci. USA, 1976, 73, 4174-4178.

Le plasmide pOP203 (UV5) contient une séquence d'ADN longue de 205 paires de bases comprise entre les deux sites de restriction EcoRI, EcoRI et comprenant :

- le gène i codant pour le répresseur de l'opéron lactose, noté "lac i" dans les figures,

- le promoteur-opérateur lactose portant les mutations L8 et UV 5 (lacL8/UV5), noté "P/O lac" dans les figures (la mutation L8/UV5 rend le système insensible à la repression catabolique)

- la séquence d'ADN codant pour la séquence S/D de l'opéron lactose noté "$SD^Z$" dans les figures,

et

- la partie du gène lacZ codant pour les huit premiers acides aminés du gène de la $\beta$-galactosidase.

On notera que ce plasmide comporte notamment un site AluI  au niveau de l'extrémité 5' du promoteur-opérateur.

Après digestions successives par les enzymes de restriction et fixation des extrémités appropriées, un fragment HindIII-EcoRI long de 95 paires de bases est obtenu à partir de pOP203(UV5).

Ces étapes consistent essentiellement en les étapes de :

- digestion EcoRI

- digestion partielle AluI,

- fixation d'une extrémité HindIII (lieur Hind III et digestion Hind III),

- digestion AluI,

- fixation d'une extrémité EcoRI (lieur EcoRI et digestion $EcoR_1$). Le fragment obtenu contient le promoteur-opérateur lacL8/UV5 dépourvu de son extrémité 5', noté "petit P/O lac" sur les figures et la région d'ADN correspondant à l'extrémité 5' non traduite de l'ARNm de l'opéron lactose et incluant la séquence Shine et Dalgarno ($SD^Z$).

Le plasmide plac2 résulte de l'insertion dans le plasmide pBR322 au niveau des sites HindIII 29 et EcoRI 4361 du fragment obtenu ci-dessus.

Les différentes étapes de construction de ce plasmide plac2 sont représentées schématiquement sur la figure 4a.

c) Construction du plasmide p46-6 (figure 4b)

Le plasmide p46-6 résulte de l'insertion dans le plasmide pBR322 ouvert au niveau du site HindIII 29 d'un fragment HindIII-EcoRI issu du plasmide plac2 contenant le promoteur-opérateur petit P/O lac et la séquence SD$^Z$ et d'un fragment EcoRI-HindIII issu du plasmide p40-4 contenant la séquence codant pour le précurseur de l'hGH.

A partir de plac2, on isole à nouveau par digestions HindIII et EcoRI le fragment de 95 paires de bases contenant le promoteur-opérateur lac L8/UV5 et la région d'ADN correspondant à l'extrémité 5' non traduite de l'ARNm de l'opéron lactose et incluant la séquence S/D. Ce fragment HindIII-EcoRI et le fragment EcoRI-HindIII isolé de p40-4 ——— par digestions HindIII et EcoRI et qui contient le gène codant pour le précurseur de l'hGH sont insérés dans pBR322 au niveau du site HindIII 29 pour obtenir un nouveau plasmide, appelé p46-6.

Les principales étapes de la construction du plasmide p46-6 sont représentées schématiquement sur la figure 4b.

Ce plasmide p 46-6 comporte 5139 paires de bases.

1.2. Construction du plasmide p126-2

Le plasmide p126-2 est construit à partir des plasmides p46-6 et p119-2.

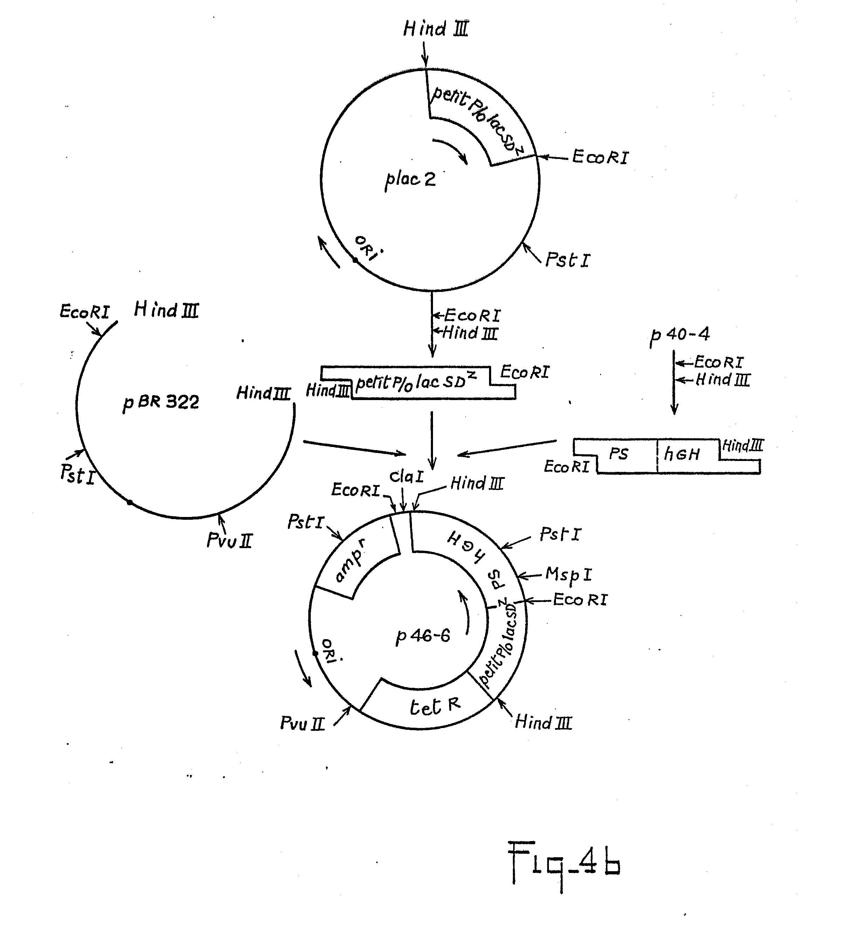

a) Construction du plasmide p119-2 (figure 5)

Le plasmide p119-2 est construit à partir du plasmide pOP203 (UV5) duquel on isole un fragment HindIII-HindIII contenant le promoteur-opérateur P/O lac, la séquence SD$^Z$ et l'extrémité 5' du gène lacZ par digestion PvuII, fixation d'une extrémité appropriée (lieur HindIII) et digestion HindIII. Ce fragment est inséré dans le plasmide pBR322 ouvert en son site HindIII 29 et fournit le plasmide p115-12. A partir du plasmide p115-12 résultant on prépare un fragment HindIII - AluI de 160 paires de bases contenant le promoteur-opérateur P/O lac et la

séquence SD$^z$ par digestions successives EcoRI, HindIII, AluI (partielle).

Ce fragment HindIII-AluI est cloné dans le plasmide pBR322 au niveau de ses sites HindIII 29 et PvuII 2066.

Les différentes étapes de fabrication de ce plasmide sont représentées schématiquement sur la figure 5.

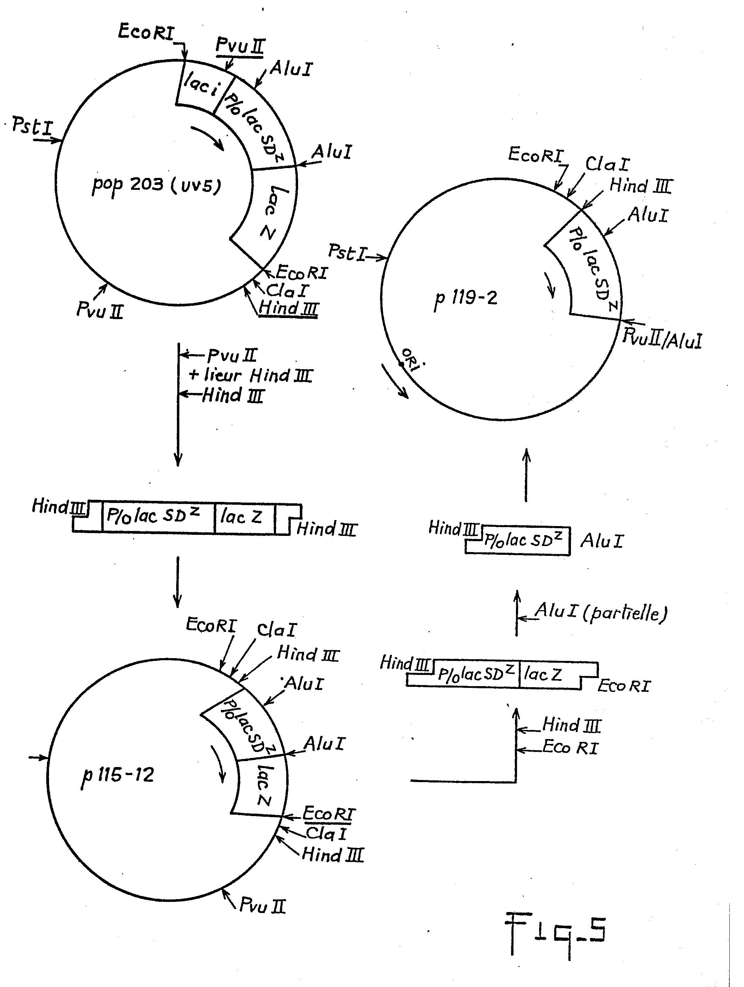

b) Construction du plasmide p126-2 (cf. figure 6)

Ce plasmide résulte de l'insertion dans le plasmide pBR322, ouvert au niveau des sites HindIII 29 et EcoRI d'un fragment EcoRI modifié-HindIII, issu du plasmide p46-6, contenant la séquence d'ADN codant pour le précurseur de l'hGH et d'un fragment PvuI-EcoRI issu du plasmide p119-2 contenant le promoteur-opérateur P/O lac et la séquence SD$^z$. Le fragment EcoRI modifié-HindIII est obtenu par digestion EcoRI, puis formation d'une extrémité franche à l'aide de l'ADN polymérase de Klenow et digestion HindIII (partielle).

Le fragment PvuII-EcoRI est obtenu à partir du plasmide p119-2 par digestion EcoRI, puis digestion PvuII.

Les différentes étapes de préparation du plasmide p126-2 sont représentées schématiquement sur la figure 6.

Le plasmide p126-2 comporte 5200 paires de bases.

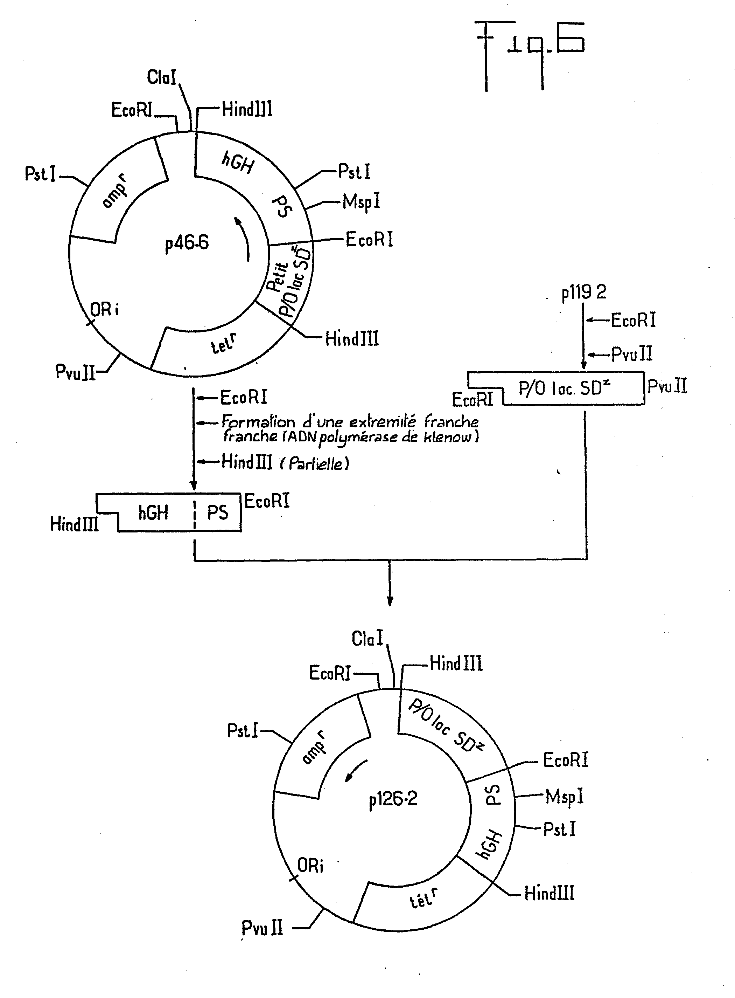

c) Construction du plasmide p149-147 (cf. figure 7)

Un fragment MspI-PstI de 200 paires de bases est préparé à partir du plasmide p46-6. Il contient la portion de la séquence d'ADN codant pour le précurseur de l'hGH comprise entre les sites de restriction MspI et PstI, tels que représentés sur la figure 1. Ce fragment est obtenu à partir du plasmide p46-6 par digestions successives avec EcoRI, PstI et MspI.

Deux fragments d'ADN sont obtenus à partir du plasmide p126-2; ces fragments sont :

- un fragment PstI-EcoRI de 947 paires de bases contenant le promoteur-opérateur lacL8/UV5 et la séquence SD$^z$ obtenu par digestions successives PstI et EcoRI;

- un fragment PstI-PstI de 4027 paires de bases contenant toute la portion de la séquence d'ADN codant pour l'extrémité carboxy-terminale de l'hGH à partir du site PstI indiqué sur la figure 1, obtenu par digestion PstI.

On prépare enfin un oligonucléotide double brin ayant pour structure :

5' AATTATGGCTACCGGTCT

3'TACCGATGGCCAGAGC

Les trois fragments d'ADN ci-dessus et l'oligonucléotide sont liés entre eux pour former le plasmide p149-147; à cet effet, on met en présence 200 ng du fragment PstI-PstI, 250ng du fragment PstI-EcoRI, 50ng du fragment MspI-PstI et 4ng de l'oligonucléotide double brin synthétique phosphorylé à l'extrémité de 5' et hybridé.

L'incubation est faite dans 20 µl de volume final pendant 18 heures à 10°C puis la réaction est arrêtée par addition de 0,8 µl d'EDTA, 0,5 M (pH 8).

Le mélange est ensuite ajusté à 100 µl, par addition de 70 µl d'H$_2$O et 10 µl de Tris-HCl (pH 7,6).

Ce mélange est utilisé pour transformer la souche d'E.coli C1024. Les colonies résistantes à l'ampicilline sont testées pour la production d'hGH par RIA, ainsi que pour la résistance à la tétracycline par étalement sur les boîtes de milieu L-agar, contenant 20 µg/ml de tétracycline.

Parmi les plasmides recombinants se trouve un plasmide comprenant une séquence composite selon l'invention. Ce plasmide p149-147 est caractérisé de la façon suivante :
- les enzymes EcoRI et ClaI le coupent en un seul point.
- l'enzyme PstI le coupe en deux points (deux fragments, l'un de 4027 paires de bases (pb) et l'autre de 1165 pb sont obtenus).
- l'enzyme HindIII le coupe en deux points, produisant deux fragments,l'un de 4360 pb et l'autre de 832 pb.
- les enzymes HindIII/PvuII utilisés simultanément génèrent 5 fragments de tailles différentes (2334 pb, 2026 pb, 487 pb, 314 pb, et 32 pb) parmi lesquels le fragment de 314 pb HindIII-PvuII qui contient le promoteur-opérateur lacL8/UV5, la séquence d'ADN composite et le début du gène hGH.

La séquence du plasmide a été confirmée par la méthode de SANGER et al., 1977, Proc. Natl. Acad. Sci., USA, 74, 5436-5467.

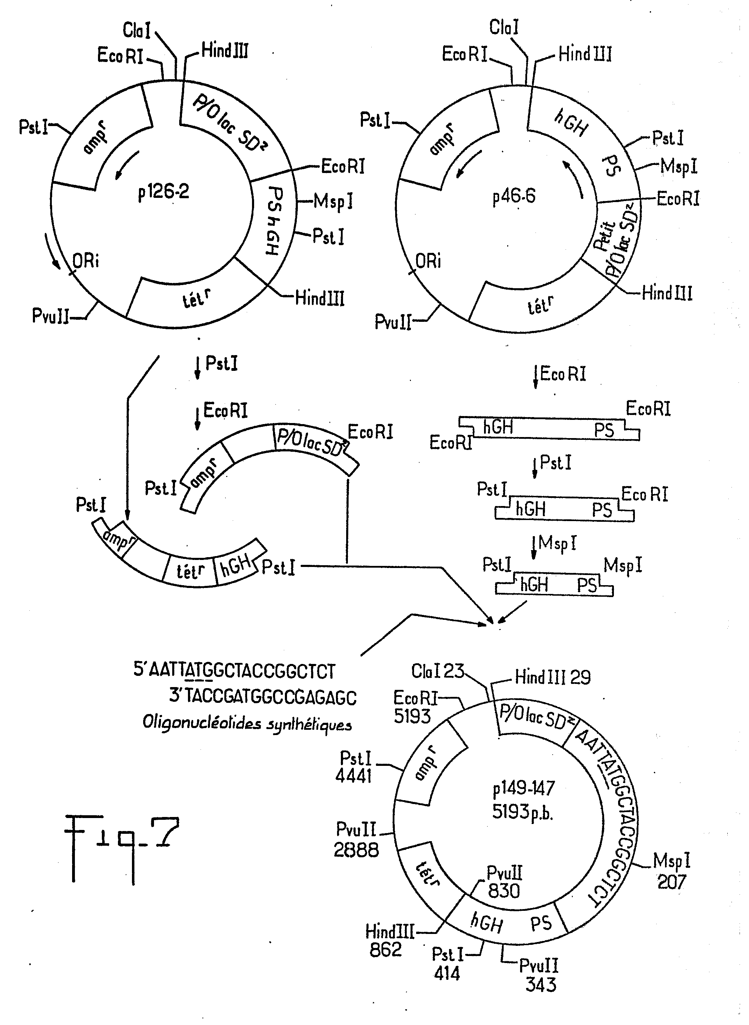

La structure de l'ARNm obtenu par transcription de la nouvelle séquence d'ADN du plasmide p149-147 est décrite dans la figure 8 où:

- la séquence 1 correspond à l'extrémité 5' non traduite de l'ARNm qui contient :

  . le site de fixation du répresseur de l'opéron lactose

  . et la séquence de Shine et Dalgarno (S/D) de l'opéron lactose

- la séquence 2 correspond à la transcription de l'oligonucléotide double brin synthétique.

- la séquence 3 correspond à la transcription du gène codant pour la pré-hGH amputée de ses quinze premiers nucléotides.

- AUG est le codon d'initiation de la traduction.

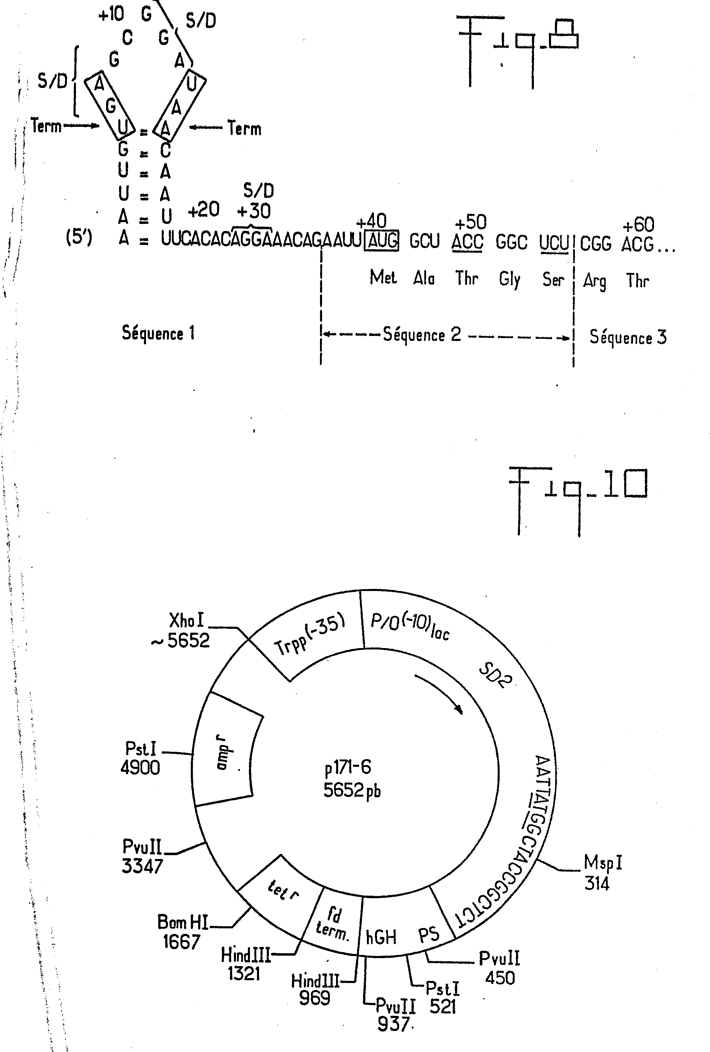

EXEMPLE 2 : CONSTRUCTION DU PLASMIDE p169-30 (figure 9)

En opérant d'une manière similaire à celle décrite dans l'exemple 1, on prépare le plasmide p169-30qui diffère du plasmide p149-147 en ce qu'il comporte un fragment supplémentaire de 352 paires de bases codant pour le terminateur de transcription du phage fd isolé du plasmide pLBU3, et inséré au niveau du site HindIII situé en aval du gène codant pour l'hGH (R. GENTZ et al., Proc. Natl. Acad. Sci., USA, 1981, 78, 4936-4940). Ce plasmide p169-30est obtenu à partir du plasmide p149-147 par insertion dans celui-ci, au site HindIII cité ci-dessus, du fragment d'ADN HindIII-HindIII du plasmide pLBU3.

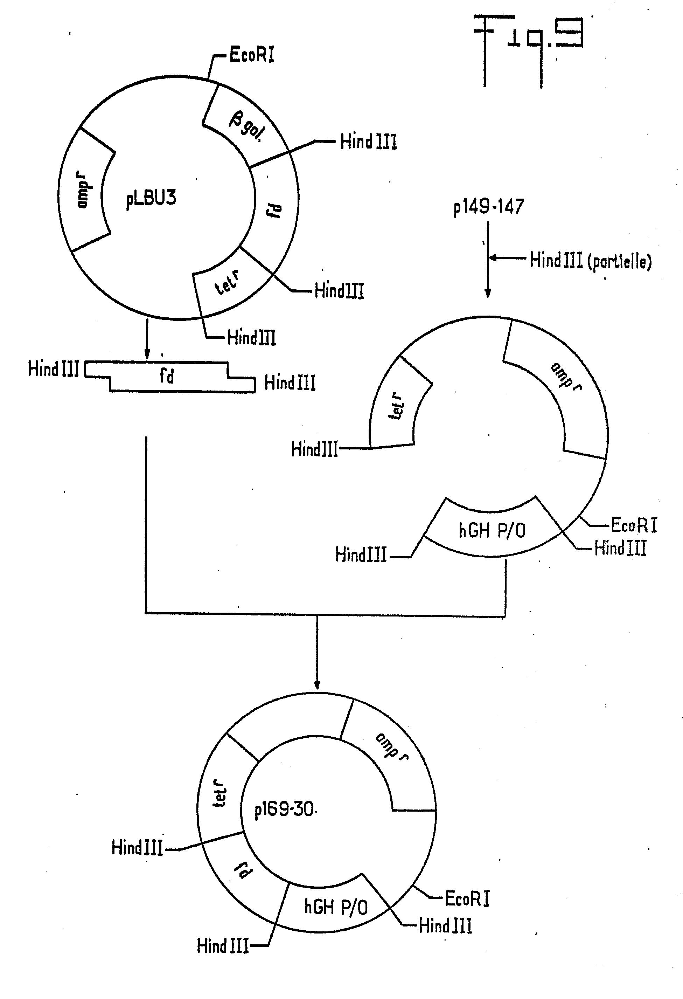

EXEMPLE 3 : CONSTRUCTION DU PLASMIDE p171-6 (figure 10)

En opérant d'une manière similaire à celle décrite dans l'exemple 1, on prépare le plasmide p171-6 qui diffère du plasmide p169-30 en ce qu'il comporte en lieu et place du promoteur-opérateur lac L8/UV5 un promoteur hybride trp-lac UV5 (promoteur "tac") (H.A. DE BOER et al., Proc. Natl. Acad. Sci., USA, 1983, 80, 21-25). La figure 9 présente la carte des sites de restriction du plasmide p171-6 ainsi obtenu.

EXEMPLE 4 : PRODUCTION ET CARACTERISATION DE l'hGH

a) Production :

Le promoteur-opérateur lac L8/UV5, présent sur un plasmide "multicopies" tel que pBR322 ou l'un des ses dérivés (par ex. p149-147),fait s'exprimer de manière constitutive le gène de l'hGH dans une lignée "normale" d'E.coli.

Des cellules d'E. coli C1024 transformées par le plasmide p149-147 ou l'un de ses dérivés (par exemple: p169-30, p171-6) sont mises en culture toute une nuit en milieu L avec 50 μg/ml d'ampicilline (ou 20 μg/ml de tétracycline dans le cas de p149-147), à 37°C, avec agitation. Cette culture sert à inoculer (inoculum à 0,5%) un autre milieu L et la nouvelle culture, après incubation, à 37°C avec agitation, est celle utilisée pour la production d'hGH.

b) Caractérisation de l'hGH

1 ml de la culture préparée comme indiqué ci-dessus est mise à incuber en milieu L à 37°C, avec agitation (jusqu'à ce qu'on obtienne une absorbance (D.O) de 3,0 à 600 nm).

Une première moitié de la culture, soit 0,5 ml, est centrifugée et le lysat total (LT) est récupéré à partir du culot.

Le culot de cellules est mis en suspension dans 0,5 ml d'une solution contenant : 15 g pour 100 ml de saccharose, 30 mM de Tris-HCl (pH 8,0), 1 mM d'EDTA.

Lyse des cellules : 20 μl d'une solution concentrée de lysozyme (10 mg/ml) sont ajoutés à la suspension cellulaire et le mélange est incubé dans la glace pendant 10 min. On y ajoute alors 100 μl d'une solution à 5 g pour 100 ml de polyoxyéthylène 20 cétyl-éther (Brij-58) (dans un tampon Tris-HCl 0,1 M, pH 7,2), 100 μl de $MgSO_4$ (1M), 200 μl d'une solution à 1 g pour 100 ml de désoxycholate (dans un tampon Tris-HCl 0,1 M, pH 8) et 80 μl d'une solution à 10 mg/ml de désoxyribonucléase. La lyse totale des cellules est produite après une heure d'incubation du mélange dans la glace.

L'autre moitié de la culture est également centrifugée, le milieu de culture est retiré et le culot de cellules est mis en suspension soigneusement dans la même solution que pour le lysat total (LT).

Après 10 min à température ambiante, la suspension cellulaire est à nouveau centrifugée, et le surnageant "saccharose" (SS) est conservé. Le culot est mis en suspension dans 0,5 ml d'eau froide puis centrifugé à 2°C et le surnageant aqueux (SA) est mis de côté. Le nouveau culot de cellules intactes est mis en suspension dans la même solution (saccharose, EDTA et Tris-HCl) et lysé (LC) de la même manière que pour le lysat total (LT).

l'hGH est dosée par RIA (dosage radioimmunométrique, Institut Pasteur Production, France) sur les fractions LT, SS, SA et LC. LOCALISATION DE l'hGH PAR DOSAGE RIA (en ng/ml de culture sur milieu L pour une absorbance à 600nm égale à 3,0) sur la souche E. coli C1024 transformée par l'un des plasmides suivants :

| PLASMIDES | SS | SA | LC | LT |
|---|---|---|---|---|
| p149-147 | 72 | 405 | 52 | 350 |
| p169-30 | 74 | 425 | 48 | 360 |
| p171-6 | 300 | 2050 | 100 | 800 |

Les résultats figurant dans ce tableau montrent clairement que la plus grande partie de l'hormone produite se trouve dans les fractions de choc osmotique SS (surnageant saccharose) et SA (surnageant aqueux) ce qui prouve que l'hGH est sécrétée dans l'espace périplasmique des cellules d'E. coli.

c) Conditions de croissance pour une production plus élevée :

Les résultats indiqués ci-dessus sont d'autant plus remarquables qu'ils sont obtenus en l'absence d'une induction qui aurait permis une dérépression complète de l'opérateur lactose et qu'aucun antibiotique n'a été ajouté à la culture (il est en effet connu que l'addition d'ampicilline lorsque les plasmides portent un gène codant pour la béta-lactamase permet d'obtenir un nombre élevé de copies du plasmide).

Des résultats plus élevés de 50% ont été également obtenus avec la souche d'E.coli K 12-1061.

## REVENDICATIONS

1. Séquence d'ADN composite caractérisée en ce qu'elle est constituée de son extrémité 5' vers son extrémité 3' par :

a) la séquence d'ADN naturelle codant pour l'extrémité 5' non traduite de l'ARN messager de l'opéron lactose d'E.coli incluant la séquence de Shine et Dalgarno ;

b) une séquence d'ADN ayant la structure 5' AATTATGGCTACCGGCTCT 3';

c) la séquence d'ADN naturelle correspondant à l'extrémité carboxy-terminale du peptide signal de l'hGH comptée à partir du seizième nucléotide constitutif du peptide signal.

2. Vecteur recombinant caractérisé en ce qu'il comporte une séquence d'ADN composite selon la revendication 1, placée entre un promoteur bactérien et le gène codant pour l'hGH.

3. Vecteur selon la revendication 2, caractérisé en ce qu'il est le plasmide ayant les caractéristiques du plasmide introduit dans la souche E.coli déposée à la Collection Nationale de Cultures de Micro-organismes sous le No. I-533.

4. Cellule procaryote transformée par un vecteur selon l'une des revendications 2 ou 3.

5. Souche bactérienne selon la revendication 4, caractérisée en ce qu'il s'agit d'une bactérie de la famille des Enterobacteriaceae.

6. Souche bactérienne selon la revendication 5, caractérisée en ce qu'il s'agit d'une souche d'Escherichia coli.

7. Procédé d'obtention de l'hormone de croissance humaine caractérisé en ce que la protéine est obtenue par culture d'un micro-organisme recombiné selon l'une des revendications 5 ou 6, suivie de l'extraction de l'hormone de la cellule, par exemple par choc osmotique et de son isolement par purification du chocat.

## Fig. 1

```
                              MspI
5' ATG GCT ACA GGC TCC┐CGG ACG TCC CTG CTC CTG GCT TTT GGC CTG CTC TGC CTG
   ----------------------------------------------------------------------------
   TAC CGA TGT CCG AGG GCC TGC AGG GAC GAG GAC CGA AAA CCG GAC GAG ACG GAC
   M   A   T   G   S   R   T   S   L   L   L   A   F   G   L   L   C   L
  -26     -24     -22

   CCC TGG CTT CAA GAG GGC AGT GCC TTC CCA ACC ATT CCC TTA TCC AGG CTT TTT
   ----------------------------------------------------------------------------
   GGG ACC GAA GTT CTC CCG TCA CGG AAG GGT TGG TAA GGG AAT AGG TCC GAA AAA
   P   W   L   Q   E   G   S   A   F   P   T   I   P   L   S   R   L   F
                           -1  1                                         10

   GAC AAC GCT ATG CTC CGC GCC CAT CGT CTG CAC CAG CTG GCC TTT GAC ACC TAC
   ----------------------------------------------------------------------------
   CTG TTG CGA TAC GAG GCG CGG GTA GCA GAC GTG GTC GAC CGG AAA CTG TGG ATG
   D   N   A   M   L   R   A   H   R   D   V   H   Q   L   A   F   L     Y
                                       20                             PstI

   CAG GAG TTT GAA GAA GCC TAT ATC CCA AAG GAA CAG AAG TAT TCA TTC CTG CAG
   ----------------------------------------------------------------------------
   GTC CTC AAA CTT CTT CGG ATA TAG GGT TTC CTT GTC TTC ATA AGT AAG GAC GTC
   Q   E   F   E   E   A   Y   I   P   K   E   Q   K   Y   S   F   L   Q
       30                                      40

   AAG CCC CAG ACC TCC CTC TGT TTC TCA GAG TCT ATT CCG ACA CCC TCC AAC AGG
   ----------------------------------------------------------------------------
   TTG GGG GTC TGG AGG GAG ACA AAG AGT CTC AGA TAA GGC TGT GGG AGG TTG TCC
   N   P   Q   T   S   L   C   F   S   E   S   I   P   T   P   S   N   R
               50                              60

   GAG GAA ACA CAA CAG AAA TCC AAC CTA GAG CTG CTC CGC ATC TCC CTG CTG CTC
   ----------------------------------------------------------------------------
   CTC CTT TGT GTT GTC TTT AGG TTG GAT CTC GAC GAG GCG TAG AGG GAC GAC GAG
   E   E   T   Q   Q   K   S   N   L   E   L   L   R   I   S   L   L   L
                           70                          80

   ATC CAG TCG TGG CTG GAG CCC GTG CAG TCC CTC AGG AGT GTC TTC GCC AAC AGC
   ----------------------------------------------------------------------------
   TAG GTC AGC ACC GAC CTC GGG CAC GTC AAG GAG TCC TCA CAG AAG CGG TTG TCG
   I   Q   S   W   L   E   P   V   Q   F   L   R   S   V   F   A   N   S
                           90                                          100

   CTG GTG TAC GGC GCC TCT GAC AGC AAC GTC TAT GAC CTC CTA AAG GAC CTA GAG
   ----------------------------------------------------------------------------
   GAC CAC ATG CCG CGG AGA CTG TCG TTG CAG ATA CTG GAG GAT TTC CTG GAT CTC
   L   V   Y   G   A   S   D   S   N   V   Y   D   L   L   K   D   L   E
                                       110

   GAA GGC ATC CAA ACG CTG ATG GGG AGG CTG GAA GAT GGC AGC CCC CGG ACT GGG
   ----------------------------------------------------------------------------
   CTT CCG TAG GTT TGC GAC TAC CCC TCC GAC CTT CTA CCG TCG GGG GCC TGA CCC
   E   G   I   Q   T   L   M   G   R   L   E   D   G   S   P   R   T   G
               120                             130

   CAG ATC TTC AAG CAG ACC TAC AGC AAG TTC GAC ACA AAC TCA CAC AAC GAT GAC
   ----------------------------------------------------------------------------
   GTC TAG AAG TTC GTC TGG ATG TCG TTC AAG CTG TGT TTG AGT GTG TTG CTA CTG
   Q   I   F   K   Q   T   Y   S   K   F   D   T   N   S   H   N   D   D
               140                             150

   GCA CTA CTC AAG AAC TAC GGG CTG CTC TAC TGC TTC AGG AAG GAC ATG GAC AAG
   ----------------------------------------------------------------------------
   CGT GAT GAG TTC TTG ATG CCC GAC GAG ATG ACG AAG TCC TTC CTG TAC CTG TTC
   A   L   L   K   N   Y   G   L   L   Y   C   F   R   K   D   M   D   K
                           160                             170

   GTC GAG ACA TTC CTG CGC ATC GTG CAG TGC CGC TCT GTG GAG GGC AGC TGT GGC
   ----------------------------------------------------------------------------
   CAG CTC TGT AAG GAC GCG TAG CAC GTC ACG GCG AGA CAC CTC CCG TCG ACA CCG
   V   E   T   F   L   R   I   V   Q   C   R   S   V   E   G   S   C   G
                           180

   TTC 3'
   -------
   AAG
   F
   191
```

2/9

0251842

Fig. 2

Fig. 3

Fig. 4a

Fig. 4b

Fig. 5

6/9

Fig.6

Fig-7

Fig-9

```
         +10  G      S/D
             G   G
      S/D   G    A
           A      U
Term →  [A]      [A] ← Term
        [G]      U
         U = A
         G = C
         U = A
         U = A
         A = U  +20    +30  S/D
(5')     A = UUCACACAGGAAACAGAAUU [AUG] GCU ACC GGC UCU │ CGG ACG...
                                       Met Ala Thr Gly Ser │ Arg  Thr

         Séquence 1        ←─── Séquence 2 ────→ │ Séquence 3
```

Fig-10

Fig.9

0251842

9/9

0251842

Office européen
des brevets

**RAPPORT DE RECHERCHE EUROPEENNE**

Numéro de la demande

EP  87  40  1248

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 020 147  (THE REGENTS OF THE UNIVERSITY OF CALIFORNIA) <br> * Revendications * | 1-7 | C 12 N  15/00 <br> C 12 P  21/02 <br> C 12 N   1/20 // <br> (C 12 N   1/20 <br> C 12 R   1:19 ) |
| Y | EP-A-0 111 814  (MOLECULAR GENETICS) <br> * Page  67,  ligne 12 - page 69, ligne 4; figure 8 II * | 1-7 | (C 12 P  21/02 <br> C 12 R   1:19 ) |
| Y | PROCEEDINGS OF THE NATIONAL ACADEMY OF SCIENCES OF THE USA, vol. 82, no. 13, juillet 1985, pages 4306-4310, Washington, US; S. SEKINE et al.: "Cloning and expression of cDNA for salmon growth hormone in ESCHERICHIA COLI" <br> * Page 4308, tableau I * | 1 | |
| Y | NATURE, vol. 281, 18 octobre 1979, pages 544-548, Macmillan Journals Ltd; D.V. GOEDDEL et al.: "Direct expression in ESCHERICHIA COLI of a DNA sequence coding for human growth hormone" <br> * En entier * | 1-7 | DOMAINES TECHNIQUES RECHERCHES (Int. Cl.4) <br><br> C 12 N |
| A | EP-A-0 154 539  (ELI LILLY & CO.) <br><br> ---          -/- | | |

Le présent rapport de recherche a été établi pour toutes les revendications

| Lieu de la recherche <br> LA HAYE | Date d'achèvement de la recherche <br> 06-10-1987 | Examinateur <br> PULAZZINI A.F.R. |
|---|---|---|

Office européen
des brevets

**RAPPORT DE RECHERCHE EUROPEENNE**

| Catégorie | Citation du document avec indication, en cas de besoin, des parties pertinentes | Revendication concernée | CLASSEMENT DE LA DEMANDE (Int. Cl.4) |
|---|---|---|---|
| Y | BIOTECHNOLOGY, vol. 4, no. 1, janvier 1986, pages 51-55, New York, US; M. MATTEUCCI et al.: "Alkaline phosphatase fusions: a tag to identify mutations that result in increased expression of secreted human growth hormone from E. COLI" * En entier * | 1 | |

## DOCUMENTS CONSIDERES COMME PERTINENTS

-----

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 06-10-1987 | PULAZZINI A.F.R. |

Office européen des brevets

Numéro de la demande: **0251842**

*8740243.7*

# DECLARATION SELON LA REGLE 28, PARAGRAPHE 4
# DE LA CONVENTION SUR LE BREVET EUROPEEN

Le demandeur a informé l'Office européen des brevets que, jusqu'à la publication de la mention de la délivrance du brevet européen ou jusqu'à la date à laquelle la demande est rejetée, retirée ou réputée retirée, l'accessibilité au(x) micro-organisme(s) identifié(s) ci-dessous, visée au paragraphe 3 de la règle 28 de la Convention sur le brevet européen, ne peut être réalisée que par la remise d'un échantillon à un expert.

## IDENTIFICATION DES MICRO-ORGANISMES

Numéros d'ordre des dépôts :  *CNCM      I- 533*

OEB Form 1165 07.81 f